Europäisches Patentamt

European Patent Office

Office européen des brevets

(11) Publication number: **0 133 346**
A2

# EUROPEAN PATENT APPLICATION

(21) Application number: **84304490.0**

(22) Date of filing: **29.06.84**

(51) Int. Cl.⁴: **C 12 H 1/00, C 12 G 1/06**

(30) Priority: **01.07.83 GB 8317953**
**14.02.84 GB 8403880**

(43) Date of publication of application: **20.02.85**
**Bulletin 85/8**

(84) Designated Contracting States: **BE DE FR GB**

(71) Applicant: **Thomas, Keith Robert, 28 Greyhound Road, London, N18 6XW (GB)**
Applicant: **Harrison, Robert John, 145 Iseldon Road, London N7 (GB)**

(72) Inventor: **Thomas, Keith Robert, 28 Greyhound Road, London, N18 6XW (GB)**
Inventor: **Harrison, Robert John, 145 Iseldon Road, London N7 (GB)**

(74) Representative: **Brown, Kenneth Richard et al, R.G.C. Jenkins & Co. 12-15, Fetter Lane, London EC4A 1PL (GB)**

(54) **Method and apparatus for secondary fermentation and vessel containing beverage.**

(57) Where a beverage, e.g. beer, is pressurized by conventional techniques in a dispensing vessel (e.g. a barrel or a bottle) by secondary fermentation in that container using yeast, it is necessary to ensure that the yeast settles properly before the beverage is dispensed. This tends to restrict the usage of such beverage to places providing the required stable environment for settling to take place. In the present invention, the yeast is restrained, so that it cannot mix freely in the beverage but still has sufficient contact with the beverage for the fermentation to proceed. Such restraining can be achieved either by entrapping the yeast within a porous or partly porous container (3) immersed in the beverage, the porosity being such as to allow passage of the beverage but not the yeast particles, or by immobilising the yeast on a substrate 3' free or fixed within the vessel (2, 22).

- 1 -

METHOD AND APPARATUS FOR SECONDARY FERMENTATION

AND VESSEL CONTAINING BEVERAGE

This invention relates to a method and apparatus for secondary fermentation of beverages and is particularly, but not exclusively applicable to the secondary fermentation of beer.

As used herein "secondary fermentation" refers to that process applied to beverages (usually, but not always a liquid containing alcohol produced in a primary fermentation process) for the purpose of pressurising and/or maturing the liquid by fermentation within the container from which the liquid is to be dispensed to the consumer, whether this be a bulk container from which the liquid is dispensed in a number of successive operations (e.g. a beer barrel or keg) or a smaller container such as a bottle from which all of the contained liquid will normally be dispensed without reclosure.

In dispensing live beer containing active yeast for secondary fermentation, the yeast must normally be allowed to settle before a clear beer may be drawn. The yeast, throughout and after the settling period, continues to ferment residual or added sugars to produce carbon dioxide. This pressurises the container and secondary metabolites are also produced which help to mature the beer.

However since yeast cells are small, bouyant and easily resuspended there is a continual problem that the beer can remain or can again become cloudy after settling, particularly if the beer is to be further transported or used in a disturbed environment. This problem generally precludes the serving of live beer on most forms of transportation such as trains and boats. Another restriction of traditional secondary fermentation techniques is that the beer should remain undisturbed in a stable setting for some time before use, usually 12 to 48 hours, and for this some secure and stable environment is required, usually at a suitable temperature of about 13 to $14^{\circ}C$ or lower. While drinking establishments such as bars, public houses and restaurants can normally provide such conditions, temporary events such as open air festivals and many private functions frequently incur difficulties with the result that the beer remains cloudy or is easily disturbed.

Even controlled conditions are not free from problems. Although fining procedures usually result in the yeast settling there are always occasions where this does not occur for some reason and the beer wasted. Furthermore, when the yeast does settle there is inevitably a volume of suspended yeast left at the bottom of the barrel. While this volume may be small within a particular barrel the cumulative volume to a brewer producing many barrels is considerable.

Similar difficulties of resuspension of yeast can arise in the case of bottled beverages, such as live beer containing yeast, where the secondary fermentation takes place in the bottle. Here, again, it is necessary to store the bottles for a suitable period of time at an appropriate temperature to allow the yeast which has caused secondary fermentation of the beverage to settle sufficiently to allow satisfactory separation of the yeast and beverage. This separation may be performed as in the case of bottled live beers, when the beverage is dispensed from the bottle, the yeast having settled at the bottom of the bottle; even then, extreme care must be taken in decanting the clear beverage above the yeast so as to avoid remixing and thus clouding of the dispensed beer. With some other bottled beverages, e.g. champagne-type and some other sparkling wines, in which secondary fermentation takes place within the bottle, the separation is traditionally performed as part of a combined operation in which after this fermentation process is complete, the yeast having been made to settle as plug in the bottle neck, the bottle is opened, the yeast is removed, and the bottle resealed. This is a difficult and complicated operation to perform, and can only be carried out after an extended yeast-settling period if the champagne is to be properly cleared.

The present invention seeks to alleviate these problems, and to that end provides a method of secondary fermentation of a beverage within a vessel from which the beverage is ultimately to be dispensed, the method involving the use of a particulate material within the vessel, said particulate material being restrained from mixing freely in the beverage, while being allowed sufficient contact with the beverage for said secondary fermentation to proceed.

The material will normally be a microorganism, in particular a yeast, but isolated enzyme complexes may be usable.

Such restraining of the microorganism (yeast) may be achieved by keeping it entrapped within a container which has pores large enough to allow molecular diffusion of the beverage and of the gas evolved during fermentation but not large enough to allow passage of the yeast particles. In the case of beer to be subjected to secondary fermentation in bulk within a barrel, the container of yeast is inserted into the barrel of clear beer and allows release of carbon dioxide to pressurise the barrel and metabolites to mature the beer but prevents dissipation of the yeast throughout the beer.

Another approach is to keep the yeast immobilised within or attached to a relatively dense supporting material. This support will easily settle to the bottom of the dispensing vessel but still allow the yeast to remain active within the beer. Alternatively the support may be attached to a part of the vessel or be an integral part of the vessel.

The present invention also provides a vessel containing a beverage to be dispensed from the vessel and a quantity of a particulate material for acting, or which has acted, upon the beverage to produce secondary fermentation thereof within said vessel, means being provided which restrains the material from freely mixing in said beverage but which allows sufficient contact between the beverage and the material for said secondary fermentation to proceed.

In accordance with the above-mentioned alternative techniques of restraining the yeast, the restraining means may comprise the porous container or the yeast-bearing supporting material immersed in the beverage within the vessel.

Further, the present invention provides apparatus for the secondary fermentation of a beverage using a particulate material, said apparatus comprising a vessel into which the beverage is to be supplied, and from which the beverage is ultimately to be dispensed, and means for restraining the material during secondary fermentation within said vessel from freely mixing in said beverage but for allowing sufficient contact between the beverage and the material for said secondary fermentation to proceed.

Preferred embodiments of the present invention will now be described by way of example with reference to the accompanying drawings, in which:

Figure 1 shows a section through a beer barrel with yeast separated in a porous container;

Figure 2 shows the construction of a type of porous container suitable for separating the yeast;

Figure 3 shows the construction of another type of porous container suitable for separating the yeast;

Figure 4 shows the construction of a further type of porous container suitable for separating the yeast;

Figure 5 shows the construction of yet another type of porous container suitable for separating the yeast;

Figure 6 shows a section through a beer barrel having built into it a still further type of porous container suitable for separating the yeast;

Figure 7 shows a section through a beer barrel with yeast immobilized by attachment to or within independent physical support means; and

Figure 8 shows a section through a beer barrel with yeast immobilized by attachment to or within a physical support means which is an integral part of the barrel.

The initial stages of the beer production process may be identical to those normally employed in brewing. The wort is boiled with hops, cooled and fermented with yeast to produce an alcohol solution. After this primary fermentation the majority of yeast settles as a first sediment but some remains in suspension. The supernatant containing these yeast cells is removed and is fined filtered or otherwise cleared so that a clear beer is obtained.

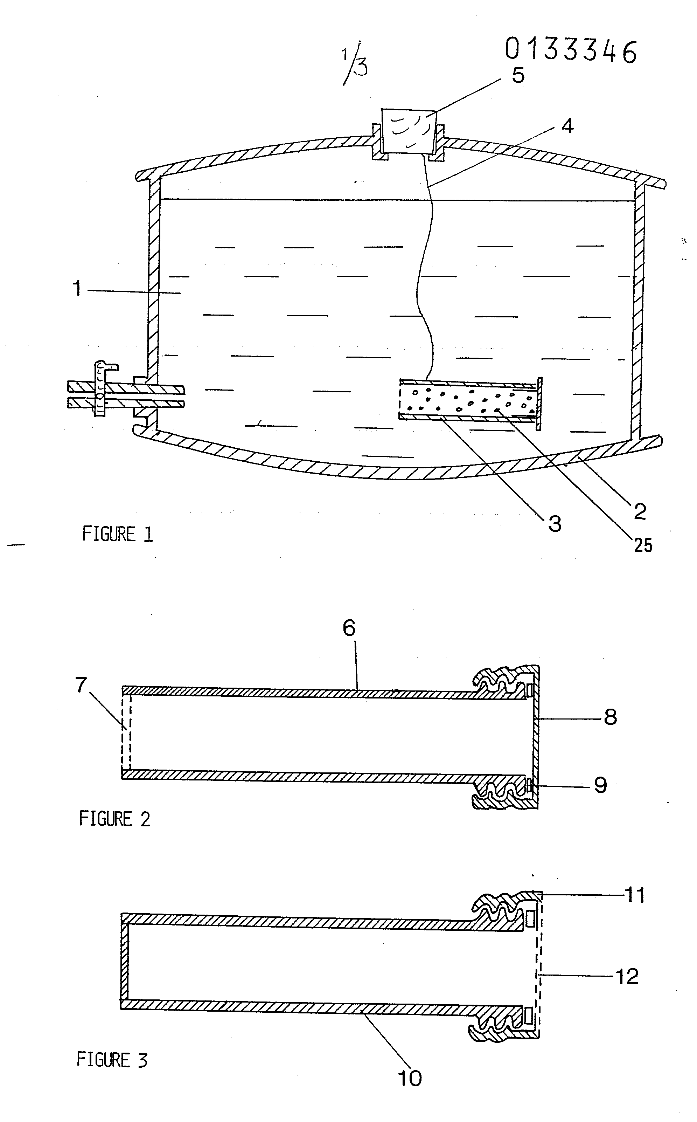

With reference first to the yeast entrapment technique, the clear beer 1 is now porued into barrels 2 or other vessels as shown in Figure 1. Fresh yeast or yeast 25 from either of the sediments produced is placed within a porous container 3 of appropriate size and this container added to the beer in the barrel. An appropriate attachment 4 to the porous container is provided to facilitate removal of the container after use. The barrel is finally sealed by the shive 5 or other appropriate means.

This procedure allows secondary fermentation to proceed with diffusion of carbon dioxide from the yeast 25 out of the container to pressurise the barrel and of metabolites to mature the beer. Priming sugars can be included in the container 3 with the entrapped yeast or can be allowed to diffuse in from the beer. However, although the yeast cells disperse throughout the beer inside the container 3, they are unable to pass out of the container to enter and cloud the rest of the beer in the barrel and therefore provided the container 3 does not obstruct the tap 24, clear, conditioned beer can be drawn despite disturbance to the barrel.

An added advantage of this procedure is that it facilitates changing of the type of yeast involved in the different stages of fermentation. For example a particular strain of yeast might be more suitable for secondary fermentation than primary fermentation. In particular it would allow the use of non-floculent strains in secondary fermentation.

Examples of the construction of the porous container are illustrated in Figs. 2, 3, 4, 5 and 6. A common feature of the containers illustrated in Figs. 2, 3, 4 and 6 is that they have both porous and non porous parts. While this is not essential and the entire container could be constructed of porous material it is more convenient and cheaper to include just a limited area of porous material.

Figure 2 illustrates a cylindrical container 6 comprising a portion of suitable porous material 7 set in a part of the container's non porous material. In the illustrated construction the porous portion 7 is a disc forming an end of the container. Access to the interior of the container for introducing the yeast and for cleaning the container after use is effected by an appropriate means which prevents leakage of the yeast, for example a screw cap 8 and sealing ring 9 at the other end of the container.

Figure 3 illustrates a cylindrical container 10 composed of non porous material and a screw cap 11 with suitable porous material 12 as part of its structure. In the illustrated construction this porous part 12 forms a closure disc of the cap 11.

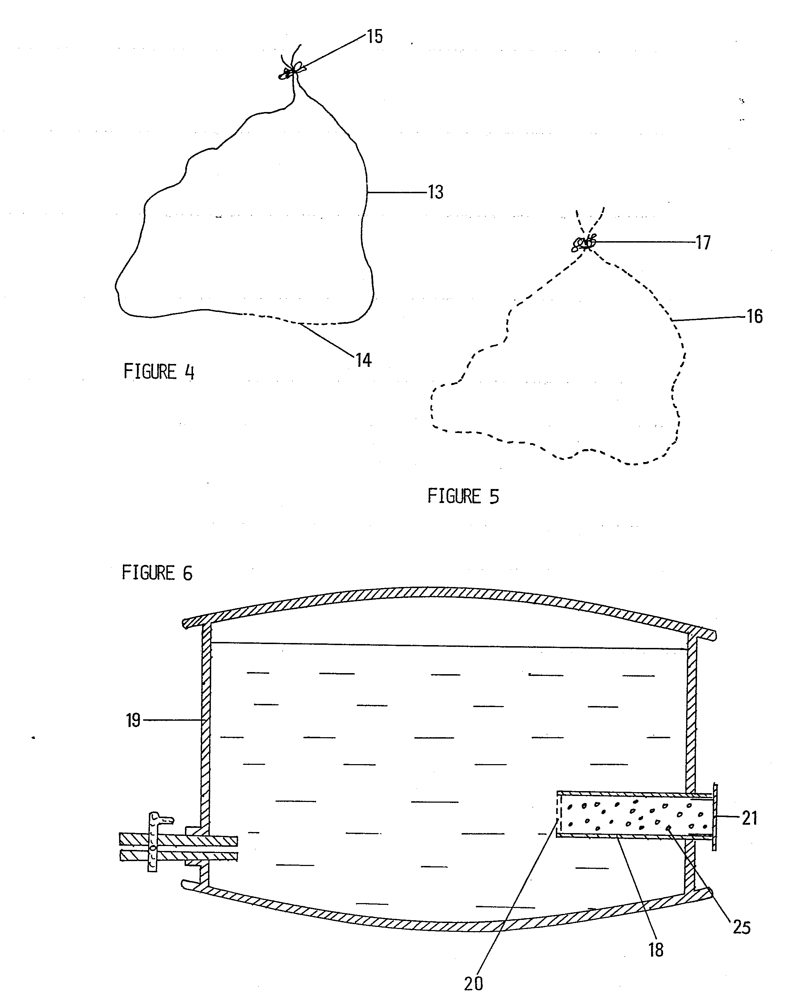

Figure 4 illustrates a container in the form of a bag made of flexible material 13 in which is set an area of suitable porous material 14. The container is tied by an appropriate means 15 once the yeast is within.

Figure 5 illustrates a container again in the form of a bag, but this time made entirely of a flexible porous material 16. Again, the bag can be sealed by an appropriate means 17.

Figure 6 illustrates a container 18 which is an integral part of the barrel 19 and has a portion of suitable porous material 20 and a means of access 21 from outside the barrel. This means of access must be capable of being securely closed when the barrel contains the beer.

Appropriate porous materials for use as the yeast entrapment containers include sintered glass discs or other mineral-based filters, cellulose or other fibre-based materials and porous plastics or other polymer-based materials, such as various rubbers.

A variety of such materials are commercially available with a range of pore sizes. A suitable pore size to prevent release of yeast cells should be reasonably small, about 0.1 to 0.2 microns due to the small cell size of yeast buds although for certain non - budding strains larger - pored materials may be suitable e.g. 0.5 to 2.0 microns. The absolute pore size does, of course, depend upon the thickness of the material, and materials with indirect pore structures such as certain plastics or sinters may permit larger pore sizes to be employed, e.g. up to about 10 microns, and even upto about 40 microns in the case of non-budding yeasts.

It is also possible to use material with even larger pores which allow easy gaseous and fluid transfer across the material but with electrostatically charged groups lining the pores to repel yeast cells showing similar charged groups on their outer surface.

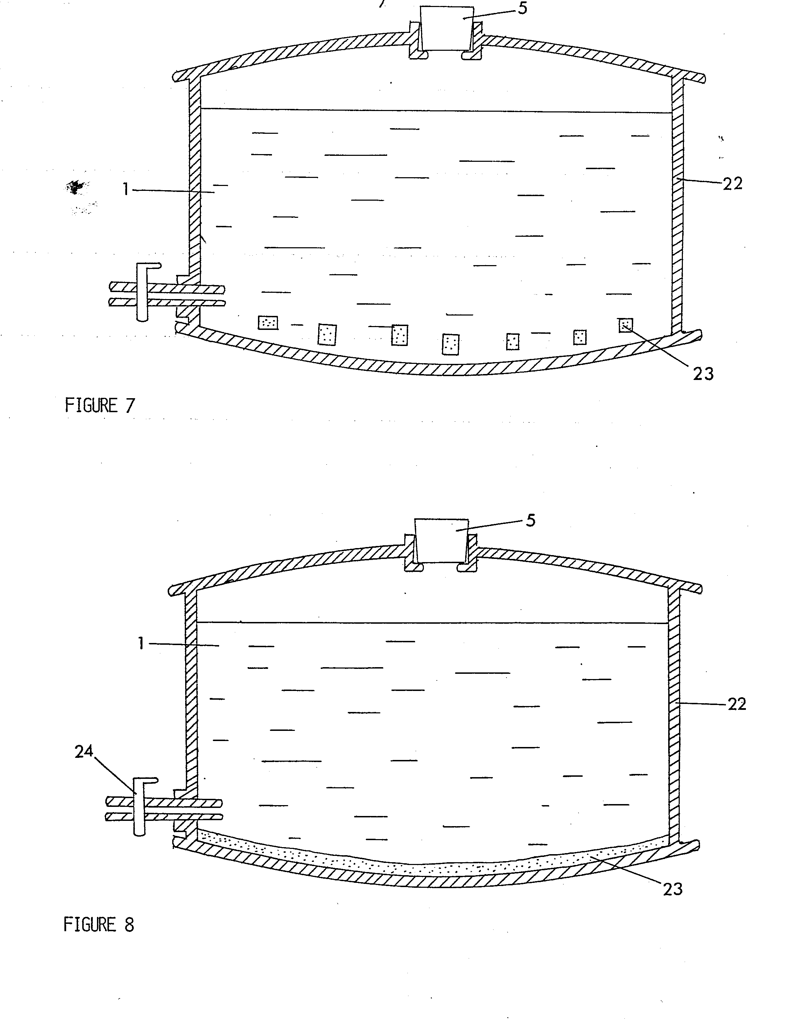

With reference now to the yeast immobilisation technique, the clear beer 1 is dispensed after its primary fermentation into suitable vessels 22 as shown in Figure 7. Fresh yeast or yeast from either of the sediments immobilised on or within an appropriate support material 23 is placed within the beer vessel. The beer may be primed with fermentable sugars as necessary for secondary fermentation and is finally sealed by the shive 5 or other means to allow gas pressure to build up.

If the support material 23 is an integral part of the vessel as shown in Figure 8 then additional procedures may be necessary to recharge the material 23 with fresh yeast for each usage of the vessel, although this would not apply to disposable plastics containers such as polypins. Suitable recharging procedures would include chemical alteration of specific bonds attaching the yeast to the support, ionic treatments to break charged associations and dissolving of an immobilising matrix.

The presence of immobilised yeasts within the container allows diffusion of carbon dioxide from the yeast into the beer. The direct contact of the yeast with the beer also allows the cells to exercise their metabolic abilities for the maturing of the beer. Priming sugars present in the beer or added as a supplement would have direct contact with the yeast cells but the cells, being immobilised on the support material 23, would be prevented from distributing throughout the beer to cause clouding.

Since secondary fermentation depends upon a supply of fermentable sugars the inclusion of these in the preparation of the immobilising agent such as agar or alginate materials will allow their ready availability to the yeast, and will result in a more immediate commencement of secondary fermentation than if the yeast were to be immobilised without the inclusion of priming sugars. In the latter case there may be considerable delay before sugars in the beer diffuse through to the yeast cells.

Since sugars included in the preparation of any entrapment agent will also tend to diffuse out into the beer, it is preferable to arrange for the fermentable sugars to be chemically or otherwise bonded to the matrix material of the immobilising agent. Yeast strains able to release lytic enzymes or other agents to sever the attachments of the sugars will thus have a steady and continuous supply of sugars for secondary fermentation. The exact means of attachment of the sugars to the matrix material will depend upon the features of the material but could include charged associations which would alter according to the pH of the surrounding medium.

The properties of an ideal immobilising system for the yeast are that it should contain and restrain the cells adequately, should prevent or restrict their growth, should be resistent to the physical forces encountered during casking and transport procedures, and should be inexpensive and non toxic.

Examples of immobilisation procedures and materials for the criteria outlined in this specification include;

1. Immobilisation within agar, agarose, alginate, carrageenan, cellulose, collagen or other polymer matrixes or using derivatives of these polymers, particularly within plastics or rubbers.

2. Adsorption through ionic, hydrogen, covalent or other bonds to an inert support material such as insoluble cellulose, ion exchange resins, mineral materials, plastics, etc.

3. Cross linkage by chemical reaction to an inert support material.

4. A combination of any of the above methods with a thin covering of a permeable material such as silicone, plastic etc.

Due to the problem of yeast budding and the subsequent release of small cells it may, in certain cases, be necessary to further treat the immobilisation material so as to prevent bud release into the

0133346

surrounding beer. Examples of such treatments include the procedure outlined in 4. above and the "hardening" of the outer surface of immobilisation materials so as to reduce the pore size of this layer.

The overall advantage of both of the foregoing entrapment and immobilisation techniques of the invention is that yeast cells would remain active but be unable to enter and cloud the beer or other beverage. Because of this the vessel, whether this be a barrel or some bulk container, or a bottle in which secondary fermentation of the beverage has taken place, could undergo considerable disturbance but clear beverage could still be dispensed.

As mentioned earlier, the technique of the invention is applicable not only to the secondary fermentation of beer but also of other alcoholic and non-alcoholic beverages.

Furthermore, it is applicable not only when the beverage is to be dispensed with the yeast still remaining in the vessel but also when at an intermediate stage after secondary fermentation is complete, the yeast has been removed and the bottle resealed, for example in making champagne and other sparkling wines. In such cases, the technique of the invention, by which the yeast is restrained from mixing freely with the wine in the bottle, facilitates the process of yeast removal from the bottle neck without any remixing of yeast with the wine.

CLAIMS:-

1. A method of secondary fermentation of a beverage within a vessel from which the beverage is ultimately to be dispensed, the method involving the use of a particulate material within the vessel, characterised in that said particulate material is restrained from mixing freely in the beverage, while being allowed sufficient contact with the beverage for said secondary fermentation to proceed.

2. A method according to claim 1 characterised in that said material is restrained by entrapment within a container immersed in the beverage, at least a portion of said container being porous so as to allow molecular diffusion of the beverage into the container, but to inhibit passage of the material particles out of the container into the bulk of beverage contained in the vessel.

3. A method according to claim 2 said container comprises a rigid hollow body and a removable closure to permit access to the interior of the container.

4. A method according to claim 2 said container comprises a bag of flexible material, at least a portion of which is porous, the bag being otherwise sealed to inhibit egress of said material from said bag into the beverage within the vessel. 5. A method according to any of claims 2 to 4 characterised in that said container is fixed to the vessel and is adapted so as to provide access to the interior of the container from outside the vessel and so as to permit access of the beverage within the vessel to the material within said container through said porous part thereof. 6. A method according to claim 1 wherein said particulate material is restrained by being immobilised on or within a support material within the vessel.

7. A method according to claim 6 characterised in that said support material is attached to the inside of the vessel.

8. A method according to any of claims 2 to 4 and 6 characterised in that said container, or support material is movable within and removable from the vessel.

9. A method according to any of claims 2 to 8 wherein the secondary fermentation utilises a fermentable sugar contained within, or carried by the container, or support material, respectively.

10. A vessel containing a beverage to be dispensed from the vessel and a quantity of a particulate material for acting, or which has acted, upon the beverage to produce secondary fermentation thereof, characterised in that said secondary fermentation is performed by a method according to any of claims 1 to 12.

11. Apparatus for the secondary fermentation of a beverage using a particulate material, said apparatus comprising a vessel into which the beverage is to be supplied, and from which the beverage is ultimately to be dispensed, characterised by the provision of means for restraining the material during secondary fermentation within said vessel from freely mixing in said beverage but for allowing sufficient contact between the beverage and the material for said secondary fermentation to proceed.

FIGURE 1

FIGURE 2

FIGURE 3

FIGURE 4

FIGURE 5

FIGURE 6

FIGURE 7

FIGURE 8